(19) Europäisches Patentamt
European Patent Office
Office européen des brevets

(11) Publication number: **0 125 368 B1**

(12) **EUROPEAN PATENT SPECIFICATION**

(45) Date of publication of patent specification: **21.06.89**

(51) Int. Cl.$^4$: **G 01 N 33/543**, G 01 N 33/557

(21) Application number: **83810563.3**

(22) Date of filing: **05.12.83**

(54) **A method of immunoassay detection by reaction-rate potentiometry using fluoride ion-selective electrode.**

(43) Date of publication of application:
**21.11.84 Bulletin 84/47**

(45) Publication of the grant of the patent:
**21.06.89 Bulletin 89/25**

(84) Designated Contracting States:
**AT BE CH DE FR GB IT LI LU NL SE**

(56) References cited:
**EP-A-0 085 276**
**WO-A-80/01081**

**ANALYTICAL CHEMISTRY, vol. 54, no. 1, January 1982, pages 68-71, Easton P.W., USA; P.W. ALEXANDER et al.: "Enzyme-linked immunoassay of human immunoglobulin G with the fluoride ion selective electrode"**

**BATTELLE TODAY, no. 29, August 1982, page 1, "Novel immunoassay system is fast and sensitive"**

(73) Proprietor: **BATTELLE MEMORIAL INSTITUTE**
**7 route de Drize**
**CH-1227 Carouge/Genève (CH)**

(72) Inventor: **Siddiqi, Iqbal**
**Tour de Champel 5**
**CH-1206 Geneva (CH)**
Inventor: **Brochot, Jean**
**Lancrans**
**F-01200 Bellegarde (FR)**

(74) Representative: **Dousse, Blasco et al**
**7, route de Drize**
**CH-1227 Carouge/Genève (CH)**

**EP 0 125 368 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention embodies a universally applicable immunoassay measuring method for use in quantitative determination of an analyte in a sample, following immunological reaction of said analyte with an antibody which specifically binds to (or is bound by) this analyte. Attached to this antibody is a catalytic unit (peroxidase) which generates signals detectable electrometrically. Inherent in this invention is the facility to monitor the signal in a continuous fashion by means of an ion-selective fluoride electrode thus allowing kinetic or reaction-rate monitoring which offers, in turn, the opportunity for rapid analysis generally applicable to immunoassay.

Background of the invention

Immunoassays are based on the specific and avid binding reaction of an antibody to an antigen. Following this reaction, the antibody bound fraction is separated from the non-antibody bound fraction and either fraction quantified by measurement of a signal generator, defined prior to the immunological reaction. Commonly immunoassays are of two major forms, quantitative *excess reagent* and *limited reagent* techniques as defined by Ekins (Ekins R.P., Radioimmunoassay and Related Procedures in Medicine, IAEA, 1977, Vienna pp. 241—268). Here reaction between the two members of the immunological binding pair can be carried out in solution with up to one of the reaction members immobilised either by adsorption or cocovalent attachment to a solid-phase matrix, essentially for ease of separation. The aim of these assays which rely on the identification of unique structural elements of the analyte by the reciprocal member of the immunological binding pair, is to estimate the amount (mass or molecular number) of particular substance of unique molecular structure in a sample.

The present invention has particular application to excess reagent assay.

Definition of some terms:

Antibody: One member of the Immunological binding pair, commonly of the Immunoglobulin G (IgG) class and represented by the abbreviation *Ab*.

Antigen: The second member of the immunological binding pair, abbreviated to *Ag*.

Label: The signal generator attached to either Ab or Ag; represented thus: *.

The reagent excess methods rely on the reagent (either Ab or Ag) present in the assay system being in excess concentration relative to the reciprocal member of the binding pair which is the analyte. The essential feature of such assays is that: the response parameter directly reflects the amount of analyte in the system and are of the following forms (1) (2):

(1) excess Ab* + Ag Ab*Ag + Ab*

(2) excess Ag* + Ab Ag*Ab + Ag*.

Here separation of antibody-bound from non-antibody bound fraction is accomplished by a range of physical, chemical or immunological techniques (e.g. Ratcliffe J. G. (1974) British Medical Bulletin *30*: 32—37), well known to persons skilled in the art.

Limited reagent assays however are systems where the reagent is present in a limited and carefully defined amount, usually less than the concentration of test substance in the system. Here the analytical measurement relies on the distribution of the reagent between sample analyte and a fixed quantity of exogenous labelled analyte and is of the following form (3):

(3) limited Ab + Ag + Ag* AbAg + AbAG* + Ag* + Ag

As above, separation of the bound from the free moieties is accomplished by well tried means and either moiety can be analysed for signal.

The above represents the conventional and widely used approaches to measuring numerous exogenous and naturally endogenous substances in the clinical investigation and detection of normal and pathological function in man. Typically endogenous factors are human plasma proteins, myeloma proteins, acute-phase reactants, tumour markers, blood clotting factors, peptide and protein hormones, steroid hormones and thyroid hormones. Exogenous factors are drugs, drug metabolites, bacterial toxins, structural properties of invading bacteria, viruses, actinomycetes, mycoplasms, fungi and organisms which cause sexually transmitted diseases. This list is readily applicable to immunoassay based on potentiometric detection of fluoride ion by reaction-rate techniques or fixed time reaction methods.

Historically, immunoassay systems have been based on radioisotopic detection techniques (e.g. Kirkham K.E. and Hunter W. M., Radioimmunoassay Methods, Churchill-Livingstone, Edinburgh, 1971) but although extremely sensitive, these systems have the unwelcome aspect of handling, storage and disposal of radioactive materials and the purchase of expensive dedicated counting apparatus.

Non-isotopic techniques have also been used whereby an enzyme is linked to one member of the immunological binding pair and the presence of the enzyme detected following the immunoassay reaction sequence (see for instance Alexander *et al.*, Anal. Chem. *54* (1982), p. 67—71 and the publication of

"BATTELLE TODAY" (29), August 1982, p. 1). The enzyme based immunoassays are attractive due to the available and considerable knowledge in detection, characterisation and preparation of antibody-enzyme conjugates and the inherent sensitivity of a signal generator (enzyme) which produces a number of signal units per molecule of generator (i.e. acts as a catalyst). These enzyme immunoassay systems exist as both limited and excess reagent immunoassays. Engvall and Perlmann (Immunochemistry *8*; 871—874, 1971) were the first to utilise and coin the acronym enzyme-linked immunosorbent assay (ELISA) which is the major example of reagent excess enzyme immunoassay. Since then, numerous forms of enzyme immunoassays have been developed (see Ishikawa E. *et al.,* Enzyme immunoassay, Igaku-Shoin, Tokyo, 1981) and have been the subject of a number of patents in this area (e.g. U.S. Patents: 3,654,090; 3,791,932; 3,839,153; 3,850,752; 3,879,262) each describing methods where an enzyme is coupled to the analyte or reagent. For instance, an analyte is immunoreacted with an excess of a substrate-bound first partner member after which an excess of an enzyme labelled second partner is added, whereby following reaction one of the products is immobilised and either the soluble or insoluble fraction estimated by means of enzyme activity.

Other types of labels have been used to monitor the bound or non-bound fraction in immunoassay. Fluorescence labels using quenching (U.S. Patent 3,996,345) and enhancement techniques (U.S. Patent 3,901,654), electron spin resonance (U.S. Patent 3,850,578) and chemiluminescence (GB Patent 1,548,741 and Canada Patent 1,116,079) have been used. However most current non-isotopic methods rely on the use of optically clean surfaces and optically pure solutions. Biological samples are generally not optically pure and can contain particulate matter of a proteinaceous or lipaemic nature which interferes optically. Fluorescent or fluorogenic assays have advantages over enzyme-chromogenic immunoassays (Murachi T., Enzyme Immunoassay, Igaku-Shoin, Tokyo 1981, pp. 5—13) being potentially more sensitive. However these assays are subject to errors due to fluoresence quenching, light scattering and high fluorescent background due to the nature of complex biological samples such as serum, plasma or urine. Other references pertaining to the invention are WO—A—8 001 081; DE—A—3 010 462; DE—A—2 824 742 and Arzneimittel Forsch. Band 28, No 11A (1978) L. Schrott "Enzymimmunoassay", p. 1934—40.

Thus there still exists a need for a safe, rugged, readily applicable detection method for general use in immunoassay signal generation. Radioisotopic techniques have the best sensitivity but have inherent dangers in their use. Optical assay techniques are readily interfered with due to the diverse nature of biological samples. However we have developed a universally applicable detection method which is not inherently hazardous to the operator, requires no specialized or expensive equipment of reagent handling and storage devices, and the signal is not nonspecifically altered by normal components of the biological specimen under analysis. More precisely, in this method which comprises using an enzyme marker in a specific reactant partner for a bound analyte, the detection of the enzyme signal involves measuring catalytically generated specific ionic species the amount of which is quantitatively related to the presence of the marker and the analyte. This technique relies on the known phenomenon that some fluoro-compounds are oxidised by $H_2O_2$ in the presence of peroxidase with the stoichiometric disruption of a fluorine carbon bond to give fluoride ions which are measured electrometrically with a specific fluoride sensitive electrode. This is described in Clinical Chemistry, 1982, *28*, 1962, and has been applied to the detection of glucose and cholesterol in a previous patent (U.S. Patent 4,353,983 and WO 80/01081). The present invention is applicable to excess reagent immunoassay.

According to the invention for measuring a polyvalent analyte antigen (Ag) involving peroxydase linked to a substrate (1) in a 'Sandwich-immunobinding" of the type "Peroxydase-Antibody" (Ab2)/Antigen (Ag)/Antibody (Ab1)-Substrate (1), the determination is effected as summarized in annexed claim 1. In brief, this method comprises the following steps:

a) adding a liquid sample of analyte (Ag) to a first reaction container provided with a substrate (1) carrying an antibody (Ab1) and performing a first incubation (1) in which a selective coupling of the antigen (Ag) with the antibody (Ab1) occurs;

b) removing the remaining liquid sample from the substrate (1) and container;

c) adding thereto a peroxidase solution which comprises a known excess of a peroxidase (POD)-antibody (Ab2) labelled complex and effecting a second incubation (II):

d) removing the solution from the container but leaving the substrate with bound Ab1/Ag/Ab2—POD immunological pair, introducing into said containers a fluoride selectively sensitive electrode, adding thereto hydrogen peroxide and a fluorocompound (RF) solution whereby a peroxidase catalyzed splitting of the fluorine into $F^-$ ion occurs;

e) reading the voltage from the electrode, this being the measuring signal of the marker relating to the amount of antigen (Ag) to be measured in the sample.

The method of the invention is illustrated in the formula below:

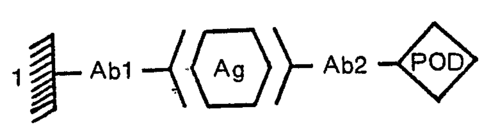


+ $H_2O_2$ + RF → $F^-$ + RO + $H_2O$

in which the vertical hatched line indicates the substrate, Ab1 is the first antibody, Ag is the analyte, Ab2 is the second antibody, POD is the enzyme marker and RF is the fluorocompound, preferably a fluoro-phenol or fluoroaniline as in US—A—4,353,983.

Brief Description of the Drawings

The following examples will better illustrate the practical aspects of the invention with reference to the annexed drawing in which:

Fig. 1 illustrates the experimental reaction-rate curves taken from example 1 which shows $F^-$ variation calculated from the corresponding electrode potential and plotted in ng/l against time for samples of different human IgG concentrations. The rate curves 1—7 shown represent human IgG concentrations of 0, 30, 100, 300, 1000, 3000 and 10,000 ng/ml respectively.

Fig. 2 is a graph obtained by plotting the mean $dF^-/dt$ values of the rate curves of Fig. 1 versus the Log of the corresponding human IgG concentrations.

Fig. 3 illustrates the experimental reaction-rate curves taken from example 2 which shows $F^-$ variation calculated from the corresponding electrode potential and plotted against time for samples of different human alphafetoprotein (AFP) concentrations. The rate curves 1—7 shown represent human AFP concentrations of 0, 5, 12.8, 32, 80, 200 and 500 ng/ml respectively.

Fig. 4 is a graph obtained by plotting the $dF^-/dt$ values of the rate curves of Fig. 3 versus the Log of the corresponding human AFP concentrations.

Fig. 5 illustrates the experimental reaction-rate curves taken from example 3 which shows $F^-$ variation calculated from the corresponding electrode potential and plotted against time for samples of different human placental lactogen (HPL) concentrations. The rate curves 1—8 represent HPL concentrations of 0, 0.24, 1, 3.9, 15.6, 62.5, 250 and 1000 ng/ml respectively.

Fig. 6 is a graph obtained by plotting the $dF^-/dt$ values of the rate curves of Fig. 5 versus the Log of the corresponding HPL concentrations.

In order to demonstrate the subject invention, the following assays were carried out. These assays are offered by way of illustration and are not to be construed as limiting.

Example 1

Determination of human IgG

Reagents:

1.a) Phosphate buffered saline (PBS) pH 7.4 was prepared by dissolving potassium hydrogen orthophosphate 7 mM (1.22 g), potassium dihydrogen orthophosphate 3 mM (0.408 g) and sodium chloride 150 mM, (8,77 g) in double distilled water.

1.b) Coating buffer pH 9.6 was prepared by dissolving sodium carbonate 15 mM (2.94 g) and sodium bicarbonate 35 mM (1.59 g) in double distilled water.

1.c) Assay buffer: 1% vol/vol normal goat serum (Scottish Antibody production Unit, Lanarkshire, Scotland) in PBS.

1.d) Wash buffer: 0.3% wt/vol bovine serum albumin (Sigma Chemical Co., St. Louis, MO, USA) and 0.05% vol/vol Tween 20 (Merck-Darmstadt, W. Germany) in PBS.

1.e) Blocking solution: 1% wt/vol. bovine serum albumin in PBS.

1.f) Goat immunoglobulin to human IgG (Scottish Antibody Production Unit, Lanarkshire, Scotland) was diluted 1/100 with counting buffer for use.

1.g) Human γ-globulin, Cohn-fraction II (Serva Feinbiochemica, Heidelberg, W. Germany) was used as standard. Working standards of 0.03, 0.1, 0.3, 1.0, 3.0 and 10 μg per ml were prepared by appropriate dilution in assay buffer.

1.h) Peroxidase conjuncted rabbit immunoglobulins to human IgG-γ-chains (Dakopatts, Copenhagen, Demark) were diluted 1/40 in the assay buffer for use.

1.i) Organo-fluoro compound solution: 4-Fluorophenol (Aldrich-Europe, Beerse, Belgium) 0.25% wt/vol in 240 mM acetate buffer and 87 mM sodium chloride pH 5.5.

1.j) Colorimetry solution: Orthophenylenediamine hydrochloride (Fluka AG, Buchs, Switzerland) 20 mg was dissolved in 10 ml of 0.1 M citrate buffer pH 4.5.

1.k) Hydrogen peroxide: 30% commercial hydrogen peroxide solution (Merck, Darmstadt, W. Germany) was diluted to 50 mM in distilled water.

Method:

A 24 wells Linbro tissue culture plate (Flow Laboratories, Ayrshire, Scotland) was used. The wells were coated by pipetting 1 ml of diluted goat antiserum (solution 1.f) and left overnight at 4°C the plate was emptied by aspiration and washed three times with 1.5 ml of wash buffer per well. The wash buffer was left 5 minutes between successive washes in order to reduce non-specific adsorption. The wells were then filled with 1.5 ml of blocking solution and left 10 minutes at room temperature. The wells were then emptied by aspiration and small adhering droplets were released by shaking the plate vigorously. Two series of wells were then filled with 1 ml of IgG working standards (solution 1.g) and a blank solution. The plate was then incubated for 90 minutes at room temperature. The wells were emptied by aspiration and washed three times with 1.5 ml of wash buffer. The wells were then filled with peroxidase conjugated

rabbit antiserum (solution 1.h) and incubated for 90 minutes at room temperature. The wells were emptied and washed three times with 1.5 ml of wash buffer as before.

In one series, the wells were filled with 1 ml of fluoro-phenol solution (solution 1.i). Miniature teflon coated magnetic bars were then gently dropped in each well. The plate was then placed on a magnetic stirrer and the stirring was started in a controlled manner to avoid spilling the solutions from the wells.

The final analytical reactions involving $F^-$ ions generation were then carried out in each well successively in the following manner: 20 µl of 50 mM hydrogen peroxide solution (solution 1.k) was injected in the well and the fluoride ion-selective electrode (type 96—09 Orion research) was then immediately inserted in the solution.

This electrode had a reference electrode combined therewith but any other fluoride ion-selective electrode with separate reference electrode can also be used. The electrode can be connected to a suitable voltmeter and in the present invention, a Corning EEL 112 Digital Research pH-meter from Corning Scientific Inc., Medfield, Mass. USA, was used. After exactly 3 minutes, the electrode potential in mV (relative) was measured every successive 15 second for 180 seconds. The concentration of $F^-$ for each measurement intervals was calculated from the measured potential from the Nernst equation which in this case has the form:

$$E = E' - S \log [F^-]$$

where E' is a constant inherent to the system which is determined experimentally and which involves activity factors and the liquid junction potentials. S is the "Nernst slope" which is also a constant and is approximately 59 mV for a change of 10 units in the concentration of $F^-$ where the latter is expressed in moles/l. Graphical curves thus obtained are shown in Figure 1. The rate of $F^-$ generation was calculated by linear regression of the $dF^-/dt$ in the ascending portion of each curve. The relationship between the concentrations of IgG and rates of $F^-$ generation is shown in Figure 2, curve 2b, and the curve is thereafter used as a comparative standard template for the determination of unknown samples of IgG the analytical conditions being the same as for the determination of standards.

In order to compare the present invention with the conventional photometric measurements, the second series of wells in the plate were treated as follows: A chromogen developing solution was prepared by mixing 3.5 µl of 30% $H_2O_2$ with 10 ml of orthophenylenediamine solution (solution 1.j). This solution must be prepared fresh. 1 ml of this solution was added to each well and the plate was then incubated in the dark, at room temperature for 30 minutes. Following this, 500 µl of the solution from each well was transferred to series of test tubes containing 2 ml of 0.2 M HCl. The absorbances of these solutions were then measured at 492 nm wavelengths in a spectrophotometer. The photometric standard curve of IgG is shown as curve 2a in Fig. 2. A comparison of the two curves 2a and 2b clearly shows that the present technique is at least as sensitive as the conventional photometric technique. The present technique is however considerably faster.

## Example 2
## Determination of human Alphafetoprotein (AFP)

Reagents:

2.a) Assay buffer: Bovine serum albumin (Sigma) 0.3% wt/vol in PBS (solution 1.a)

2.b) Rabbit immunoglobulin to human AFP (Dakopatts, Copenhagen, Denmark) was diluted 1/250 in the coating buffer (solution 1.b).

2.c) AFP standards: AFPL (Behringwerk, Marburg, W. Germany) was diluted in the assay buffer to obtain 10, 30, 100, 300 and 600 ng/ml AFP working standard solution.

2.d) Peroxidase conjugated rabbit immunoglobulin to human AFP (Dakopatts, Copenhagen, Denmark) was diluted 1/125 in PBS (solution 1.a).

2.e) Wash buffer. Surfactant Tween 20 (Merck Darmstadt, Germany) 0.1 ml was added to 1 liter of assay buffer.

Method:

The method was essentially similar to the one described in Example 1. The wells of the Linbro tissue culture plate were coated with 1 ml solution of rabbit antibody to AFP (solution 2.b). After the usual washing and blocking steps as described in Example 1, two series of wells were filled with 1 ml AFP working standards (solution 2.c) and a blank solution. The plate was incubated for 90 minutes at room temperature. The wells were then emptied and washed as described earlier. 1 ml of peroxidase conjugated rabbit antibody to AFP (solution 2.d) was then added and the plate incubated for 90 minutes at room temperature. Following this the wells were again washed, and finally filled with fluorophenol solution (solution i). Potentiometric reaction-rate measurements for $F^-$ were then carried out in one series of wells as described earlier. Reaction-rate curves are shown in Fig. 3 and the calibration curves for AFP are shown in Fig. 4, curve 4b. In the second series of wells colorimetric reactions were carried as described earlier and the data are represented by curve 4a for comparison with the present technique.

Example 3

Determination of human placental lactogen (HPL)

Reagents:

3.a) Assay buffer. Bovine serum albumin (Sigma) 0.3% wt/vol and normal horse serum (Scottish Antibody Production Unit, Scotland) 2% vol/vol in PBS (solution 1.a).

3.b) Wash buffer. Bovine serum albumin (Sigma) 3 g and surfactant Tween 20 (Merck) 0.1 ml in 1 litre of PBS (solution 1.a).

3.c) Rabbit immunoglobulin to HPL (Dakopatts, Copenhagen, Denmark) was diluted 1/125 in the coating buffer (solution 1.b).

3.d) Peroxidase conjugated rabbit immunoglobin to HPL (Dakopatts, Copenhagen, Denmark) was diluted 1/125 in the assay buffer.

3.e) HPL standard solutions. HPL standard (Biodata-Serono, Rome, Italy) was diluted in the assay buffer to give 0.060, 0.240, 0.980, 3.90, 15.60, 62.50, 250 and 1000 ng/ml HPL working standards.

Method:

The method was essentially the same as described in Example 1. Two series of wells of the Linbro tissue culture plate were coated with 1 ml solution of rabbit antibody to HPL (solution 3.c). The washing and blocking steps were performed as in Example 1. The wells were filled with HPL working standards (solution 3.e) including a blank solution and the plate incubated for 90 minutes at room temperature. The wells were then emptied and washed as described earlier. 1 ml of peroxidase conjugated rabbit antibody to HPL (solution 3.d) was added and the plate incubated again for 90 minutes at room temperature. Following this the wells were washed. Finally one series of wells were filled with 1 ml of fluorophenol solution (Solution i). Potentiometric reaction-rate measurements were then carried out as described earlier (see Fig. 5). The reaction-rate curves and the calibration curve are shown in Figs. 5 and 6 respectively (see Curve 6b). Comparative colorimetric measurements were carried on a second series of wells in the usual manner and the data is shown in Fig. 6, curve 6a, for comparison with the present technique.

**Claims**

1. A method for measuring a polyvalent antigen (Ag) involving peroxidase linked to a substrate (1) in a "Sandwich-immunobinding" of the type "Peroxidase-Antibody" (Ab2)/Antigen (Ag)/Antibody (Ab1)-Substrate (1), characterized in performing the following steps:

a) adding a liquid sample of analyte (Ag) to a reaction container provided with a substrate (1) carrying an antibody (Ab1) and performing a first incubation (1) in which a selective coupling of the antigen (Ag) with the antibody (Ab1) occurs;

b) removing the remaining sample liquid from the substrate (1) and container;

c) adding thereto a peroxidase solution which comprises a known excess of a peroxidase (POD)-antibody (Ab2) labelled complex and effecting a second incubation (II);

d) removing the solution from the container but leaving the substrate with bound Ab1/Ag/Ab2—POD immunological pair, introducing into said containers a fluoride selectively sensitive electrode, adding thereto hydrogen peroxide and a fluoro-compound (RF) solution, whereby a peroxidase catalyzed splitting of the fluorine with $F^-$ ions occurs;

e) reading the voltage from the electrode, this being the measuring signal of the marker relating to the amount of antigen (Ag) to be measured in the sample.

2. A method according to claim 1, characterized in that the fluorocompound is a fluorophenol.

**Patentansprüche**

1. Verfahren zur Messung eines polyvalenten Antigens (Ag) unter Verwendung von Peroxidase, die mit einem Substrat (1) in einer "Sandwich-Immunobindung" des Typs "Peroxidase-Antikörper" (Abs)/Antigen (Ag)/Antikörper (Ab1)-Substrat (1) verbunden ist, dadurch gekennzeichnet, daß folgende Schritte durchgeführt werden:

a) eine flüssige Probe des Analyten (Ag) wird einem Reaktionsbehälter zugesetzt, der mit einem Substrat (1) versehen ist, das einen antikörper (Ab1) trägt, und es wird eine erste Inkubation (1) durchgeführt, bei der ein selektives Koppeln des Antigens (Ag) mit dem Antikörper (Ab1) erfolgt;

b) die verbleibende Flüssigprobe wird vom Substrat (1) und dem Behälter entfernt;

c) dem wird eine Peroxidaselösung zugesetzt, die einen bekannten Überschuß eines mit Peroxidase (POD)-Antikörper (Ab2) markierten Komplexes aufweist, und es wird eines zweite Inkubation (II) bewirkt.

d) die Lösung wird vom Behälter entfernt, jedoch das Substrat mit dem gebundenen Ab1/Ag/Ab2—POD immunologischen Paar belassen, in den Behälter wird eine fluoridionenselektive Elektrode eingebracht, es wird Wasserstoffperoxid und eine Lösung einer Fluorverbindung (eF) zugesetzt, wodurch eine mittels Peroxidase katalysierte Aufspaltung des Fluors in $F^-$-Ionen auftritt, und

e) die Spannung der Elektrode wird abgelesen, wobei dies das Meßsignal der Markierung ist, die der Menge an in der Probe zu messenden Antigen (Ag) entspricht.

2. Verfahren nach Anspruch 1, dadurch gekennzeichnet, daß die Fluorverbindung ein Fluorphenol ist.

**Revendications**

1. Procédé de dosage d'un antigène polyvalent (Ag) faisant intervenir un substrat support (1) relié à de la peroxydase par formation d'un complexe immunologique "sandwich" de type Peroxydase-Anticorps (Ab2)/Antigène (Ag)/Anticorps (Ab1)-Substrat (1), caractérisé en ce qu'on effectue les étapes suivantes:

a) on ajoute un échantillon d'analyte (Ag) liquide dans un récipient de réaction contenant un substrat (1) porteur d'un anticorps (Ab1) et on laisse s'effectuer une première incubation (I) pendant laquelle se forme de manière sélective un complexe entre l'antigène (Ag) et l'anticorps (Ab1);

b) on élimine le liquide provenant de l'échantillon du récipient contenant le substrat (1);

c) on ajoute à celui-ci une solution peroxidasique contenant un excédent connu d'un conjugué marqué peroxydase (POD)-anticorps (Ab2) et on laisse s'effectuer une seconde incubation (II);

d) on élimine la solution du récipient, mais on y laisse le substrat lié à la paire immunologique Ab1/Ag/Ab2—POD, on introduit dans ledit récipient une électrode sélectivement sensible aux fluorures, on ajoute aussi du peroxyde d'hydrogène et un composé fluoré (RF), en suite de quoi il se produit une scission catalysée par la peroxydase de la liaison du fluor et formation d'ions $F^-$;

e) on prend note de la tension de l'électrode, celle-ci constituant le signal fourni par le marqueur correspondant à la quantité d'antigène (Ag) à doser dans l'échantillon.

2. Procédé suivant la revendication 1, caractérisé en ce que le composé fluoré est un fluorophénol.

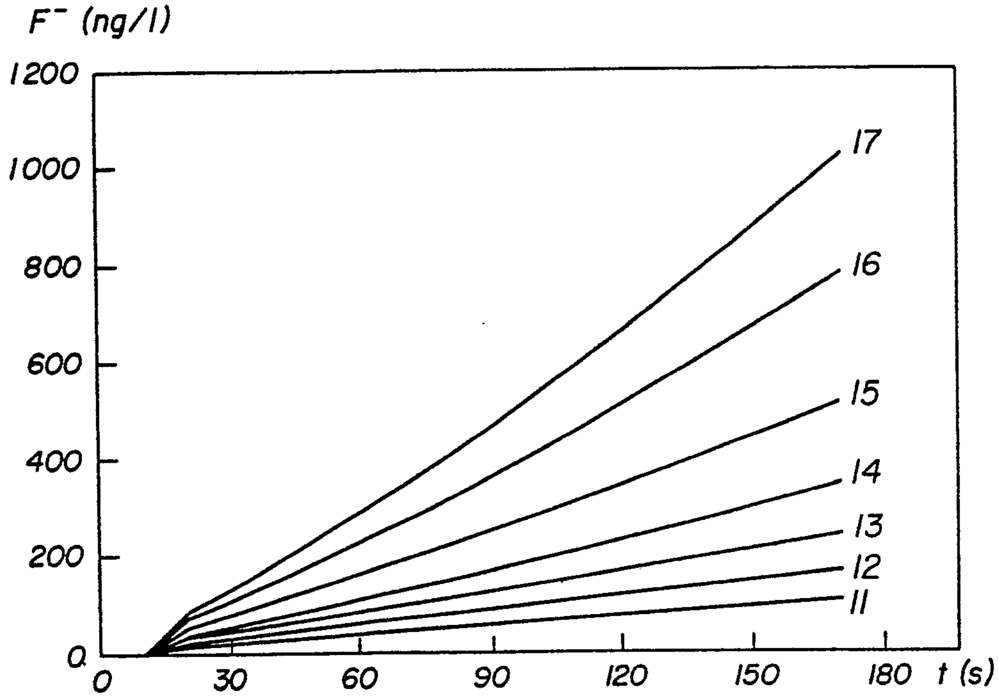
F⁻ (ng/l)
1200
1000
800
600
400
200
0
0
30
60
90
120
150
180
t (s)
17
16
15
14
13
12
11

FIG. 1

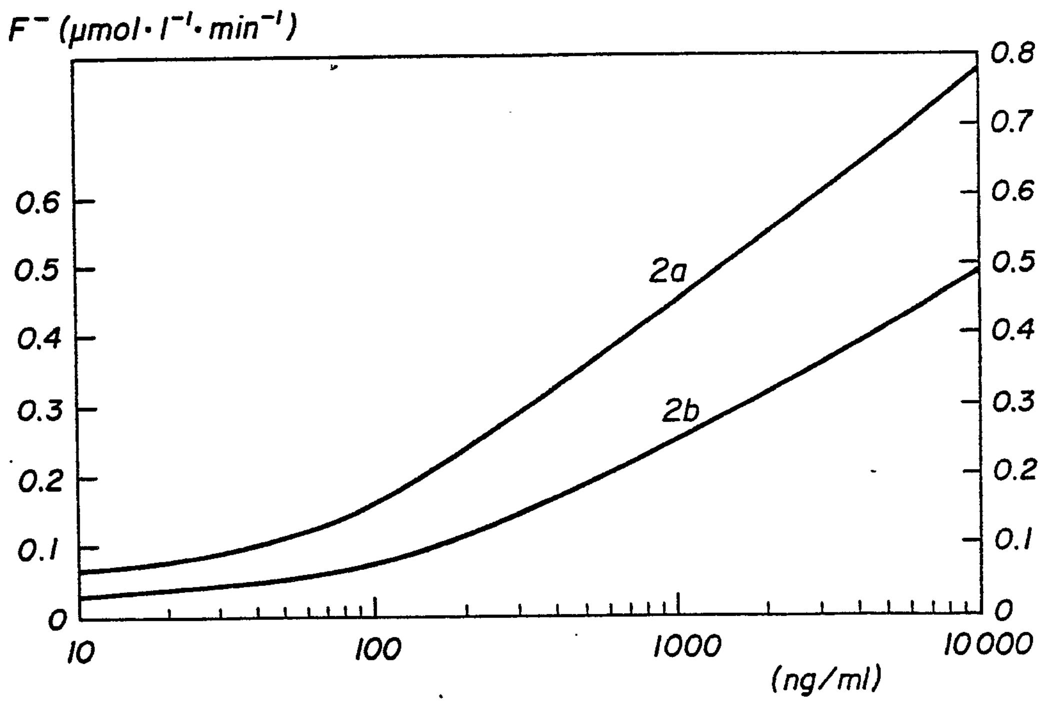
F⁻ (µmol·l⁻¹·min⁻¹)
0.6
0.5
0.4
0.3
0.2
0.1
0
0.8
0.7
0.6
0.5
0.4
0.3
0.2
0.1
0
2a
2b
10
100
1000
10 000
(ng/ml)

FIG. 2

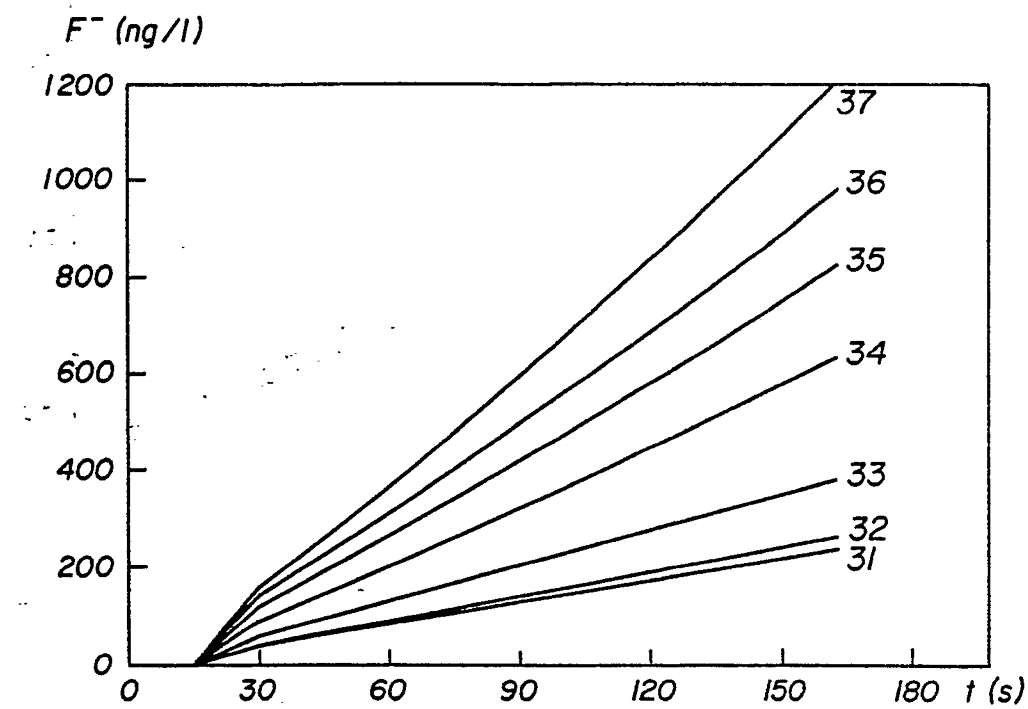
F⁻ (ng/l)
1200
1000
800
600
400
200
0
0
30
60
90
120
150
180
t (s)
37
36
35
34
33
32
31

FIG. 3

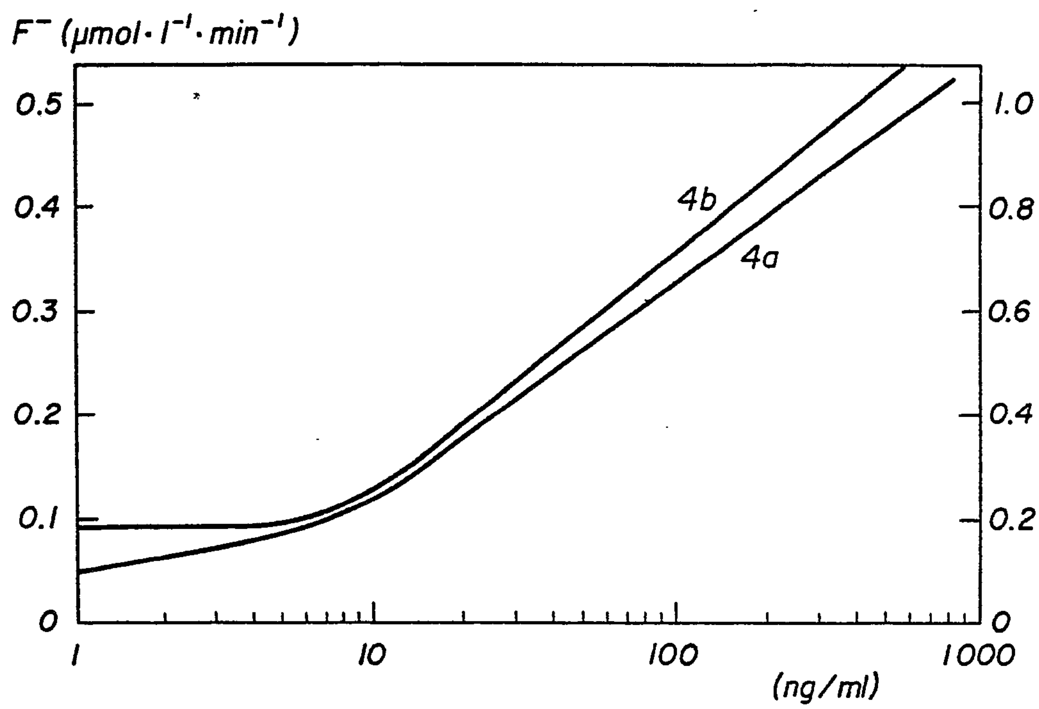
F⁻ (µmol·l⁻¹·min⁻¹)
0.5
0.4
0.3
0.2
0.1
0
1.0
0.8
0.6
0.4
0.2
0
1
10
100
1000
(ng/ml)
4b
4a

FIG. 4

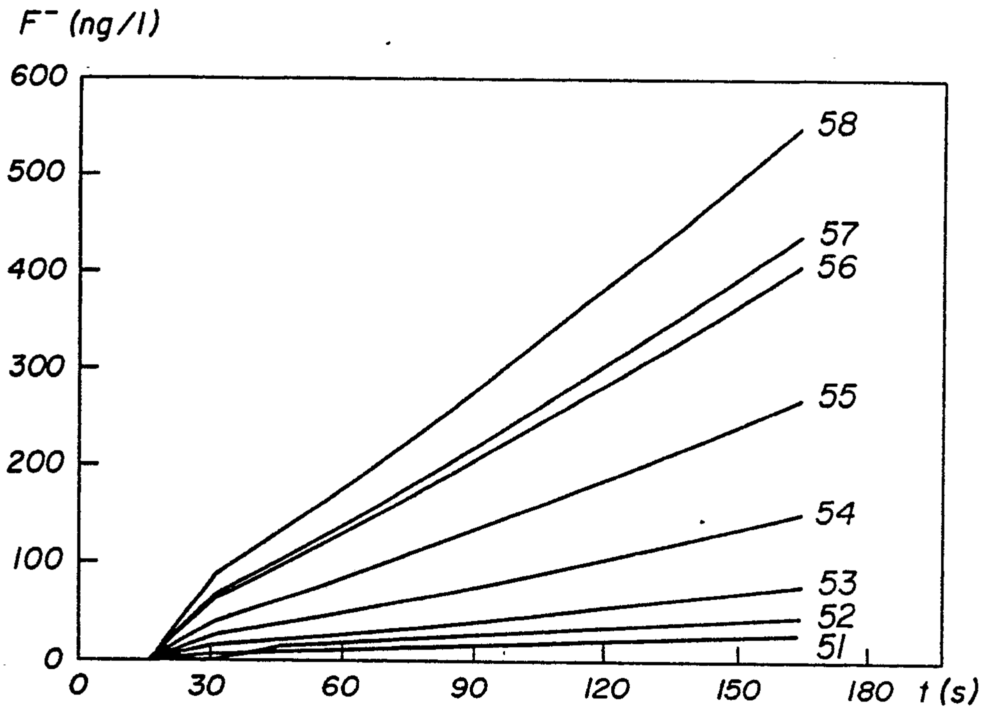
F⁻ (ng/l)
600
500
400
300
200
100
0
0
30
60
90
120
150
180
t (s)
58
57
56
55
54
53
52
51

FIG. 5

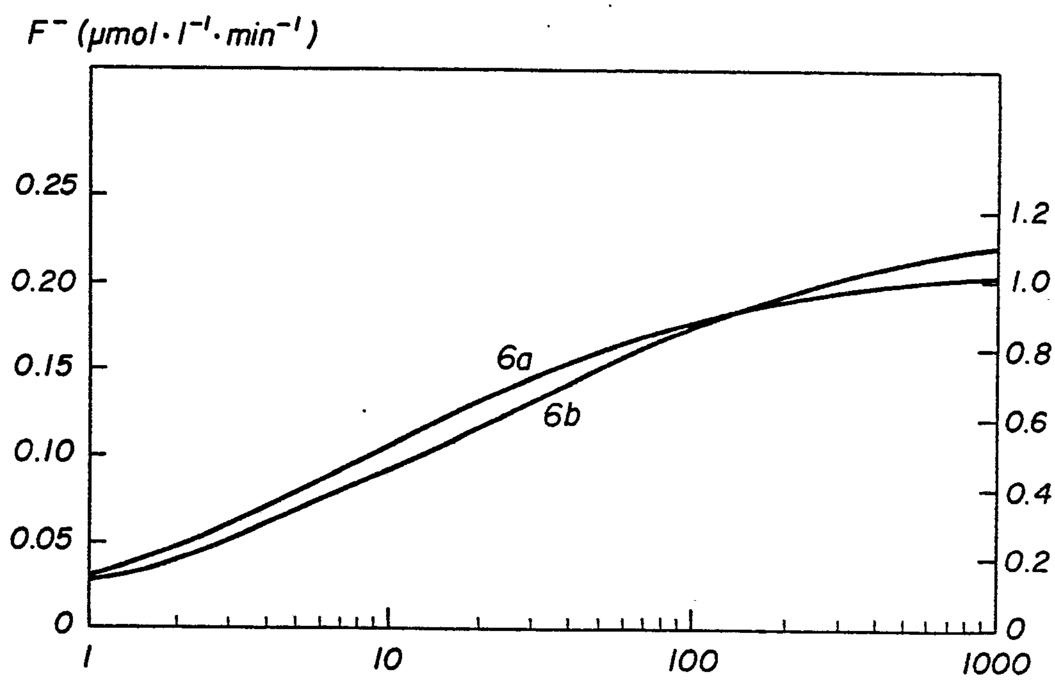
F⁻ (μmol · l⁻¹ · min⁻¹)
0.25
0.20
0.15
0.10
0.05
0
1
10
100
1000
1.2
1.0
0.8
0.6
0.4
0.2
0
6a
6b

FIG. 6